# EUROPÄISCHE PATENTSCHRIFT

(11) **EP 0 530 469 B1**
(45) Veröffentlichungstag und Bekanntmachung des Hinweises auf die Patenterteilung: **29.11.1995**
(21) Anmeldenummer: 92111741.2
(22) Anmeldetag: 10.07.1992
(51) Int. Cl.: C08F 8/14, C07C 69/96, C07D 207/27, C07D 207/404, C07D 209/48

(54) **Verfahren zur Herstellung von tert.-Butoxycarbonyl-Gruppen tragenden organischen Verbindungen**
Process for the preparation of organic compounds carrying tert.-butoxycarbonyl groups
Procédé de préparation de composés organiques portant des groupes tert.-butoxycarbonyle

(30) Priorität: 19.07.1991 DE 4124029
(43) Veröffentlichungstag der Anmeldung: 10.03.1993
(73) Patentinhaber: HOECHST AKTIENGESELLSCHAFT, 65926 Frankfurt am Main (DE)
(72) Erfinder: Eichhorn, Mathias, Dr., W-6272 Niedernhausen (DE); Buhr, Gerhard, Dr., W-6240 Königstein (DE)

(56) Entgegenhaltungen:
- EP-A- 0 102 450
- EP-A- 0 254 853
- EP-A- 0 492 254
- ANGEWANDTE CHEMIE. INTERNATIONAL EDITION Bd. 23, Nr. 4, April 1984, WEINHEIM DE , Seiten 296-297 G Höfle et al: 'A Convenient Method for the preparation of 1-(tert-Butyloxycarbonyl) pyrroles'

## Beschreibung

Die Erfindung betrifft ein Verfahren zur Herstellung von tert.-Butoxycarbonyl-Gruppen tragenden, organischen monomeren oder polymeren Verbindungen. Derartige Verbindungen finden vielfältige Anwendungen in lichtempfindlichen Beschichtungen, wie sie z.B. in den EP-A 0 102 450, EP-A 0 404 206 und EP-A 0 249 139 sowie der DE-A 38 17 010 beschrieben sind.

Bekannte Verfahren zur Einführung der tert.-Butoxycarbonyl-Gruppierung in Monomere oder Polymere gehen von Verbindungen mit Heteroatomen, wie Stickstoff oder Sauerstoff, an die ein sauer reagierendes Proton gebunden ist, aus, welche in einem inerten Lösemittel gelöst und anschließend durch Zusatz äquimolarer Mengen einer starken Base, wie Kalium-tert.-butylat, deprotoniert werden. Das gebildete Anion wird durch Zugabe von Di-tert.-butyldicarbonat, welches gegebenfalls in einem unter den Reaktionsbedingungen inerten Lösemittel gelöst ist, in die gewünschte tert.-Butoxycarbonyl-Gruppierung umgewandelt.

Nachteile dieser Verfahrensweise liegen in der heterogenen Reaktionsführung: Da das gebildete Anion in der Regel schwerlöslich ist, benötigt man große Mengen Lösemittel. Ferner muß unter Luft- und Wasserausschluß gearbeitet werden, was dieses Verfahren präparativ aufwendig macht. Die entstehenden Neben- und Abfallprodukte machen es außerdem erforderlich, die gewünschten Produkte zu isolieren und zu reinigen.

In der EP-A 0 102 450 wird ein Herstellungsverfahren beschrieben, welches durch polymeranaloge Umsetzung mit Hilfe der Phasen-Transfer-Katalyse zu den gewünschter Polymeren mit tert.-Butoxycarbonyl-Gruppierungen führt. Auch bei diesem Verfahren müssen die entstehenden Produkte durch Fällung aus der organischen Phase isoliert werden.

In der EP-A-0 492 254 wird ein Verfahren beschrieben, bei dem Maleinimid in Gegenwart einer heterocyclischen Stickstoffverbindung mit wenigstens einem tertiären Stickstoffatom mit einer etwa äquimolaren Menge Di-tert.-butyldicarbonat in einem geeigneten Lösungsmittel bei Temperaturen etwa bis zu 80 °C zur Reaktion gebracht wird. Die Herstellung anderer organischer Verbindungen, die tert.-Butoxycarbonyl-Gruppen tragen, wird dort nicht erwähnt.

Aus der EP-A-0 254 853 ist ein Verfahren zur Einführung einer tert.-Butoxycarbonylgruppe in ein Poly-p-hydroxystyrol-copolymerisat bekannt, das in Gegenwart von 4-Dimethylaminopyridin und Triethylamin in Tetrahydrofuran durchgeführt wird. Das Reaktionsprodukt wird nach Entfernung des Lösungsmittels in Methylenchlorid gelöst, mit einer Natriumbicarbonatlösung behandelt und dann in Ethylether ausgefällt. Ein Verfahren, das ohne Triethylamin allein mit katalytischen Mengen 4-Dimethylaminopyridin auskommt, ist dort nicht offenbart.
Schließlich ist auch aus Angew.Chemie. Int.Ed.Engl. 23, 296 (1984) die Umsetzung von Pyrrol mit Di-tert.-Butyldicarbonat in Gegenwart von 4-Dimethylaminopyridin bekannt. Die Acylierung des basisch reagierenden Pyrrols gelingt mit katalytischen Mengen von 4-Dimethylaminopyridin.

Aufgabe der vorliegenden Erfindung ist es, ein Verfahren vorzuschlagen, welches die gewünschten Monomeren und Polymeren in einfacher Weise bei großer Ausbeute zugänglich macht. Das Herstellungsverfahren sollte dabei breite Anwendbarkeit finden, einen geringen Anfall von Neben- oder Abfallprodukten haben, einfache Übertragbarkeit in technisch ausübbare Maßstäbe ermöglichen und, besonders im Fall der polymeren Verbindungen, eine solche Reaktionsführung bieten, daß eine Isolierung der Produkte möglichst entbehrlich ist.

Die Aufgabe wird bei einem Verfahren zur Herstellung von tert.-Butoxycarbonyl-Gruppen tragenden, organischen monomeren oder polymeren Verbindungen dadurch gelöst, daß man eine organische Verbindung, die mindestens ein Heteroatom mit einem sauer reagierenden Proton besitzt und aus der Gruppe der aliphatischen oder aromatischen Alkohole, der Amide oder Lactame ausgewählt ist, in Gegenwart von 0,01 bis 10 Molprozent, bezogen auf die umzusetzende Verbindung, eines Katalysators bei Temperaturen im Bereich von 0 bis 80°C mit Di-tert.-butyldicarbonat in einem inerten Lösungsmittel umsetzt. Die erhaltene Reaktionslösung wird zur weiteren Verarbeitung direkt verwendet oder das gebildete Produkt wird aus der Reaktionslösung durch Ausfällen in Wasser, Absaugen und Trocknen isoliert und gegebenenfalls durch Umfällen gereinigt.

Als organische Verbindungen können sowohl polymere wie monomere Verbindungen eingesetzt werden. Sie werden ausgewählt aus der Gruppe der aliphatischen oder aromatischen Alkohole, der Amide, oder Lactame.

Als polymere Verbindungen kommen beispielsweise Polyhydroxystyrol,Polyhydroxymethylstyrol oder Polybrenzkatechinmonomethacrylat sowie Polymerisate, die Styrol, Hydroxystyrol, Hydroxymethylstyrol oder Brenzkatechinmonomethacrylat als monomere Einheiten enthalten, in Frage. Vorzugsweise werden Poly-p-hydroxystyrol, Polybrenzkatechinmonomethacrylat, Copolymerisate des Brenzkatechinmonomethacrylats und Styrol oder Phenolharze, wie die bekannten Novolake, allein oder im Gemisch eingesetzt.

Als monomere Verbindungen kommen beispielsweise β-Naphthol, Brenzkatechinmonomethacrylat, Dekan-1,10-diol und 2-Pyrrolidon allein oder im Gemisch in Frage.

Als Katalysatoren dienen Verbindungen wie tertiäre Amine oder N,N-Dialkyl-4-aminopyridine, beispielsweise Diazabicyclooctan, Diazabicyclononen, Diazabicycloundecen, N,N-Dimethyl-4-aminopyridin oder Pyrrolidinopyridin. Besonders bevorzugt werden Diazabicyclooctan und N,N-Dimethyl-4-aminopyridin eingesetzt. Das erfindungsgemäße Verfahren führt in großer Ausbeute zu den gewünschten Verbindungen und erfüllt alle angeführten Anforderungen.

Erfindungsgemäß wird der Katalysator vorzugsweise im Bereich von 0,05 bis 2 Molprozent, insbesondere im Bereich von 0,1 bis 1 Molprozent, bezogen auf die umzusetzende Verbindung verwendet.

Die Umsetzung wird vorzugsweise bei Temperaturen im Bereich von 10 bis 50°C, insbesondere im Bereich von 20 bis 30°C, durchgeführt.

Die erhaltene Reaktionslösung kann direkt verwendet werden. Gegebenenfalls kann das Isolieren des Umsetzungsproduktes durch Ausfällen in Wasser, Absaugen und Trocknen erfolgen. Das Isolieren des Produktes kann aber auch so durchgeführt werden, daß man lediglich das inerte Lösungsmittel, gegebenenfalls unter vermindertem Druck, abdestilliert.

Zur Durchführung des erfindungsgemäßen Verfahrens löst oder suspendiert man das monomere oder polymere Ausgangsprodukt, das mindestens ein Heteroatom enthält, an das ein sauer reagierendes Proton gebunden ist, in einem unter den Reaktionsbedingungen inerten Lösemittel, wie zum Beispiel Tetrahydrofuran, Diethylether oder Essigsäureethylester. Als Ausgangsprodukte sind aliphatische oder aromatische Alkohole, Amide oder Lactame bzw. ein diese Gruppen enthaltendes Polymer geeignet. Man fügt der Lösung Katalysator hinzu und gibt anschließend unter mechanischem Rühren Di-tert.-butyldicarbonat, das gegebenenfalls in einem der obengenannten Lösemittel gelöst ist, hinzu.

In der Regel ist die Umsetzung vorzugsweise bei Raumtemperatur von 20 bis 30°C nach einigen Minuten bis wenigen Stunden beendet, bei Bedarf kann auch bei höherer oder tieferer Temperatur gearbeitet werden. Das Fortschreiten der Reaktion läßt sich mit einem aufgesetzten Blasenzähler anhand des bei der Reaktion entweichenden Kohlendioxids gut verfolgen. Zur Produktisolierung wird das Lösemittel, gegebenenfalls unter vermindertem Druck, abgezogen bzw. das Produkt in Wasser gefällt und erforderlichenfalls weiter gereinigt, wie durch Umkristallisieren, Umfällen, Destillation oder chromatographische Verfahren.

Der besondere Vorteil des erfindungsgemäßen Verfahrens besteht darin, daß, insbesondere bei der Umsetzung von Polymeren, keine Produktisolierung erfolgen muß: Wählt man als Reaktionsmedium ein Lösemittel bzw. Lösemittelgemisch, welches zum Aufbringen von lichtempfindlichen Schichten geeignet ist, kann die Reaktionslösung (gegebenenfalls nach Zusatz weiterer Komponenten) direkt zum Beschichten genutzt werden; die Menge des organischen Katalysators stört erfahrungsgemäß die Weiterverarbeitung sowie die reprographischen Eigenschaften nicht.

Durch die Erfindung wird erreicht, daß man in einfacher Weise und in großer Ausbeute tert.-Butoxycarbonyl-Gruppen tragende organische Verbindungen herstellen kann. Die Reaktion kann bei kaum erhöhter Umgebungstemperatur erfolgen und bisher notwendiger Luft- oder Wasserausschluß entfallen. Das erfindungsgemäße Verfahren läßt sich deshalb auch leicht in einen großtechnischen Maßstab übertragen.

Die Erfindung wird anhand der folgenden Beispiele weiter beschrieben, ohne sie hierauf zu beschränken. Sie zeigen die Ausführung des erfindungsgemäßen Verfahrens.

### Beispiel 1:

In 35 ml Tetrahydrofuran löst man 10 g kommerziell erhältliches Poly-(4-hydroxystyrol), z.B. MARUZEN Resin M, gibt 200 mg N,N-Dimethyl-4-aminopyridin (DMAP) hinzu und tropft anschließend eine Lösung von 20 g Di-tert.-butyldicarbonat in 15 ml Tetrahydrofuran zu. Man rührt 1 Stunde bei Raumtemperatur, fällt in 1 l Wasser, saugt ab und trocknet im Vakuumtrockenschrank bei 60°C/100 mbar. Man erhält 17,5 g (96% d.Th.) eines Polymers, welches im IR-Spektrum keine OH-Bande aufweist und in positiv arbeitenden, lichtempfindlichen Gemischen eingesetzt werden kann.

### Beispiel 2:

100 g kommerziell erhältliches Poly-(4-hydroxystyrol), MARUKA Lyncur M, Hersteller: Maruzen Petrochemical Co.Ltd., Hydroxylzahl 455, und 200 mg DMAP werden in 400 ml Tetrahydrofuran gelöst und unter magnetischem Rühren eine Lösung von 50 g Di-tert.-butyldicarbonat in 100 ml Tetrahydrofuran zugetropft. Nach beendeter Kohlendioxidentwicklung wird noch 1 Stunde nachgerührt. Die Reaktionslösung weist einen Feststoffgehalt von 22 % auf. Sie ist über mehrere Monate lagerfähig und kann direkt - nach Zusatz eines photochemischen Säurespenders, Farbstoffen, Lösemitteln etc. - zum Beschichten von vorbehandelten Aluminiumträgern verwendet werden, wodurch man eine positiv arbeitende Offsetdruckplatte erhält.

Eine Probe der Reaktionslösung wurde in Wasser gefällt; das erhaltene Polymer weist eine Hydroxylzahl von 267 auf.

### Beispiel 3:

10 g kommerziell erhältliches Poly-(4-hydroxystyrol), MARUKA Lyncur M, Hersteller: Maruzen Petrochemical Co.Ltd., Hydroxylzahl 455, und 50 mg Diazabicyclooctan (DABCO) werden in 60 ml Tetrahydrofuran gelöst und der Lösung unter magnetischem Rühren eine Lösung von 5,45 g Di-tert.-butyldicarbonat in 10 ml Tetrahydrofuran zugetropft. Nach beendeter Kohlendioxidentwicklung wird in H₂O gefällt, abgesaugt und bei 50°C/100 mbar getrocknet. Man erhält 11,5 g eines Polymers mit der Hydroxylzahl 270.

### Beispiel 4:

Ersetzt man in Beispiel 3 DABCO durch Diazabicycloundecen (DBU), erhält man 9,25 g eines Polymers mit der Hydroxylzahl 383.

### Beispiel 5:

Ersetzt man in Beispiel 3 DABCO durch Diazabicyclononen (DBN), erhält man 9,42 g eines Polymers mit der Hydroxylzahl 353.

### Beispiel 6:

In 40 ml Tetrahydrofuran löst man 10 g eines Brenzkatechinmonomethacrylat/Styrol-Copolymerisates (Styrolanteil 15%; Hydroxylzahl 244), gibt 50 mg DMAP hinzu und tropft dem Ganzen unter magnetischem Rühren eine Lösung von 4,4 g Di-tert.-butyldicarbonat in 10 ml Tetrahydrofuran zu. Nach beendeter CO₂-Entwicklung rührt man noch 3 Stunden nach, fällt in 1 l Wasser, saugt ab und trocknet bei 60°C/100 mbar. Ausbeute 11,3 g (94%); Hydroxylzahl 148.

### Beispiel 7:

10 g eines handelsüblichen Phenolharzes (Alnovol PN 430, HOECHST AG, Hydroxylzahl 434) und 50 mg N,N-Dimethyl-4-aminopyridin werden unter magnetischem Rühren in 40 ml Tetrahydrofuran gelöst und anschließend wird eine Lösung von 1,5 g Di-tert.-butyldicarbonat in 10 ml Tetrahydrofuran zugetropft. Nach 24 Stunden fällt man in Wasser, saugt ab und trocknet (60°C/100 mbar). Man erhält 10,5 g eines Polymers mit der Hydroxylzahl 347.

### Beispiel 8:

10 g 2-Naphthol und 50 mg DMAP werden in 25 ml Essigsäureethylester gelöst. Unter magnetischem Rühren tropft man eine Lösung von 15,1 g Di-tert.-butyldicarbonat in 20 ml Essigsäureethylester zu, rührt 5 Stunden bei Raumtemperatur und zieht das Lösemittel am Rotationsverdampfer ab. Man erhält 16,9 g (99% d. Th.) 2-tert.-Butoxycarbonylnaphthol als schwach gelbliche Kristalle vom Schmp. 71-72°C. NMR-Spektrum (60 MHz, CDCl₃): 1,6 ppm (s, 9H); 7,1-8,0 ppm (m, 7H).

### Beispiel 9:

Ersetzt man in Beispiel 8 DMAP durch DABCO, erhält man 16,5 g (98% d. Th.) 2-tert.-Butoxycarbonylnaphthol als schwach gelbliche Kristalle vom Schmp. 71-72°C.

### Beispiel 10:

In 20 ml Tetrahydrofuran löst man 5 g Brenzkatechinmonomethacrylat und 50 mg DMAP, tropft unter magnetischem Rühren 6,1 g Di-tert.-butyldicarbonat in 20 ml Tetrahydrofuran zu und rührt noch 5 Stunden. Nach Abziehen des Lösemittels erhält man 7,7 g (99% d. Th.) tert.-Butoxycarbonylbrenzkatechinmonomethacrylat als zähes Öl. NMR-Spektrum (60 MHz, CDCl₃): 1,5 ppm (s, 9H); 2,0 ppm (s, 3H); 5,7 ppm (m, 1H); 6,3 ppm (s, 1H); 7,2 ppm (s, 4H).

### Beispiel 11:

Zu einer magnetisch gerührten Suspension von 8,5 g Decan-1,10-diol und 50 mg DMAP in 30 ml Essigsäureethylester gibt man 21,8 g Di-tert.-butyldicarbonat und erhitzt zum Sieden. Nach 2 Stunden läßt man auf Raumtemperatur abkühlen und zieht das Lösemittel am Rotationsverdampfer ab. Man erhält 18,5 g (97 % d. Th.) öliges, 1,10-Di-tert.-butoxycarbonyldecandiol.

### Beispiel 12:

Zu einer Lösung von 3,8 ml 2-Pyrrolidon und 50 mg DMAP in 15 ml Essigsäureethylester wird unter magnetischem Rühren eine Lösung von 10,9 g Di-tert.-butyldicarbonat in 10 ml Essigsäureethylester getropft. Man rührt 45 Stunden bei Raumtemperatur, arbeitet wäßrig auf, zieht das Lösemittel ab und destilliert den Rückstand am Kugelrohr (100°/1 Torr). Man erhält 7,6 g (82% d. Th.) N-tert.-Butoxycarbonylpyrrolid-2-on als farbloses Öl, welches im IR-Spektrum keine NH-Bande aufweist.

### Beispiel 13 (Vergleich):

Man suspendiert 7,4 g Phthalimid und 50 mg DMAP in 20 ml Essigsäureethylester und tropft unter magnetischem Rühren eine Lösung von 11 g Di-tert.-butyldicarbonat in 15 ml Essigsäureethylester zu. Nach 1 Stunde wird die erhaltene klare Lösung eingeengt und der erhaltene farblose Feststoff (12,3 g) aus Methanol/H₂O umgefällt. Man erhält 8,3 g (68% d. Th.) N-tert.-Butoxycarbonylphthalimid vom Schmp. 90-92°C.

### Beispiel 14 (Vergleich):

Zu einer Suspension von 5 g Succinimid und 50 mg DMAP in 25 ml Essigsäureethylester tropft man unter magnetischem Rühren eine Lösung von 11 g Di-tert.-butyldicarbonat in 10 ml Essigsäureethylester. Nach Aufarbeitung erhält man 9,3 g (93% d. Th.) N-tert.-Butoxycarbonylsuccinimid als hellgelben Feststoff vom Schmp. 76-78°C.

## Patentansprüche

1. Verfahren zur Herstellung von tert.-Butoxycarbonyl-Gruppen tragenden, organischen monomeren oder polymeren Verbindungen, dadurch gekennzeichnet, daß man eine organische Verbindung, die mindestens ein Heteroatom mit einem sauer reagierenden Proton besitzt und aus der Gruppe der aliphatischen oder aromatischen Alkohole, der Amide oder Lactame ausgewählt ist, in Gegenwart von 0,01 bis 10 Molprozent, bezogen auf die umzusetzende Verbindung, eines Katalysators bei Temperaturen im Bereich von 0 bis 80°C mit Di-tert.-butyldicarbonat in einem inerten Lösungsmittel umsetzt.

2. Verfahren nach Anspruch 1, dadurch gekennzeichnet, daß man als organische Verbindung ein Polymer einsetzt.

3. Verfahren nach Anspruch 1 oder 2, dadurch gekennzeichnet, daß man als organische Verbindung ein Monomer einsetzt.

4. Verfahren nach den Ansprüchen 1 und 2, dadurch gekennzeichnet, daß man als organische polymere Verbindung Poly-p-hydroxystyrol, Polybrenzkatechinmonomethacrylat, Copolymerisate aus Brenzkatechinmonomethacrylat und Styrol oder Phenolharze allein oder im Gemisch einsetzt.

5. Verfahren nach den Ansprüchen 1 und 3, dadurch gekennzeichnet, daß man als monomere Verbindung β-Naphthol, Brenzkatechinmonomethacrylat, Dekan-1,10-diol, 2-Pyrrolidon, Phthalimid und Succinimid allein oder im Gemisch einsetzt.

6. Verfahren nach Anspruch 1, dadurch gekennzeichnet, daß man als Katalysator ein tertiäres Amin oder ein N,N-Dialkyl-4-aminopyridin verwendet.

7. Verfahren nach Anspruch 6, dadurch gekennzeichnet, daß man als Katalysator Diazabicyclooctan oder N,N-Dimethyl-4-aminopyridin verwendet.

8. Verfahren nach den Ansprüchen 6 oder 7, dadurch gekennzeichnet, daß man 0,05 bis 2 Molprozent, bezogen auf die umzusetzende Verbindung, an Katalysator verwendet.

9. Verfahren nach Anspruch 1, dadurch gekennzeichnet, daß man die Umsetzung im Bereich von 10 bis 50°C durchführt.

10. Verfahren nach Anspruch 1, dadurch gekennzeichnet, daß man die erhaltene Reaktionslösung direkt zur Weiterverarbeitung verwendet.

11. Verfahren nach Anspruch 1, dadurch gekennzeichnet, daß man das gebildete Produkt durch Ausfällen aus der Reaktionslösung in Wasser, Absaugen und Trocknen isoliert.

12. Verfahren nach Anspruch 1, dadurch gekennzeichnet, daß man das gebildete Produkt durch Abdestillieren des inerten Lösemittels, gegebenenfalls unter vermindertem Druck isoliert.

## Claims

1. A process for preparing organic monomeric or polymeric compounds carrying tert.-butyloxycarbonyl groups, wherein an organic compound, which possesses at least one heteroatom with an acidic proton, and which is selected from the group comprising aliphatic or aromatic alcohols, the amides or lactames, is reacted with di-tert.-butyl dicarbonate in an inert solvent in the presence of 0.01 to 10 mol percent, relative to the compound to be converted, of a catalyst at temperatures in the range from 0 to 80°C.

2. The process as claimed in claim 1, wherein a polymer is used as the organic compound.

3. The process as claimed in claim 1 or 2, wherein a monomer is used as the organic compound.

4. The process as claimed in any of claims 1 and 2, wherein poly-p-hydroxystyrene, poly(pyrocatechol monomethacrylate), copolymers of pyrocatechol monomethacrylate and styrene, or phenolic resins alone or as a mixture are used as the organic polymeric compound.

5. The process as claimed in any of claims 1 and 3, wherein β-naphthol, pyrocatechol monomethacrylate, decane-1,10-diol, 2-pyrrolidone, phthalimide and succinimide alone or as a mixture are used as the monomeric compound.

6. The process as claimed in claim 1, wherein a tertiary amine or an N,N-dialkyl-4-aminopyridine is used as the catalyst.

7. The process as claimed in claim 6, wherein diazabicyclooctane or N,N-dimethyl-4-aminopyridine is used as the catalyst.

8. The process as claimed in claim 6 or 7, wherein 0.05 to 2 mol percent, relative to the compound to be converted, of catalyst is used.

9. The process as claimed in claim 1, wherein the reaction is carried out in the range from 10 to 50°C.

10. The process as claimed in claim 1, wherein the resulting reaction solution is used directly for further processing.

11. The process as claimed in claim 1, wherein the product formed is isolated by precipitation from the reaction solution in water, filtration with suction and drying.

12. The process as claimed in claim 1, wherein the product formed is isolated by distilling off the inert solvent, if necessary under reduced pressure.

## Revendications

1. Procédé pour la préparation de composés organiques monomères ou polymères, portant des groupes tert-butoxycarbonyle, caractérisé en ce que l'on fait réagir un composé organique, qui possède au moins un hétéroatome comportant un proton à caractère acide et qui est choisi parmi les alcools aliphatiques ou aromatiques, les amides et les lactames, en présence de 0,01 à 10 % en moles, par rapport au composé à mettre en réaction, d'un catalyseur, à des températures dans la plage de 0 à 80°C, avec du dicarbonate de di-tert-butyle, dans un solvant inerte.

2. Procédé selon la revendication 1, caractérisé en ce que, comme composé organique, on utilise un polymère.

3. Procédé selon la revendication 1 ou 2, caractérisé en ce que, comme composé organique, on utilise un monomère.

4. Procédé selon les revendications 1 et 2, caractérisé en ce que, comme composé polymère organique, on utilise le poly-p-hydroxystyrène, le poly(monométhacrylate de pyrocatéchol), des copolymères de monométhacrylate de pyrocatéchol et de styrène ou des résines phénoliques, seules ou en mélange.

5. Procédé selon les revendications 1 et 3, caractérisé en ce que, comme composé monomère, on utilise le β-naphtol, le monométhacrylate de pyrocatéchol, le décane1,10-diol, la 2-pyrrolidone, le phtalimide ou le succinimide, seuls ou en mélange.

6. Procédé selon la revendication 1, caractérisé en ce que, comme catalyseur, on utilise une amine tertiaire ou une N,N-dialkyl-4-aminopyridine.

7. Procédé selon la revendication 6, caractérisé en ce que, comme catalyseur, on utilise le diazabicyclooctane ou la N,N-diméthyl-4-aminopyridine.

8. Procédé selon la revendication 6 ou 7, caractérisé en ce que l'on utilise de 0,05 à 2 % en moles, par rapport au composé à faire réagir, de catalyseur.

9. Procédé selon la revendication 1, caractérisé en ce que l'on effectue la réaction dans la plage allant de 10 à 50°C.

10. Procédé selon la revendication 1, caractérisé en ce que la solution réactionnelle obtenue est utilisée directement pour la suite du processus.

11. Procédé selon la revendication 1, caractérisé en ce que l'on isole le produit formé par précipitation dans de l'eau, à partir de la solution réactionnelle, séparation par filtration et séchage.

12. Procédé selon la revendication 1, caractérisé en ce que l'on isole le produit formé par élimination du solvant inerte par distillation, éventuellement sous pression réduite.
